# EUROPEAN PATENT SPECIFICATION

(11) **EP 0 432 162 B1**
(45) Date of publication and mention of the grant of the patent: **14.09.1994**
(21) Application number: 89907336.5
(22) Date of filing: 02.06.1989
(51) Int. Cl.: C12M 1/00, G01N 33/48

(54) **APPARATUS AND METHOD FOR AUTOMATED SEPARATION OF EXTRA-CHROMOSOMAL DNA FROM A CELL SUSPENSION**
APPARAT UND VERFAHREN ZUR AUTOMATISCHEN ABTRENNUNG VON EXTRACHROMOSOMALER DNS AUS EINER ZELLSUSPENSION
APPAREIL ET PROCEDE POUR LA SEPARATION AUTOMATISEE D'ADN EXTRA-CHROMOSOMIQUE CONTENU DANS UNE SUSPENSION CELLULAIRE

(43) Date of publication of application: 19.06.1991
(73) Proprietor: Uhlen, Mathias, S-752 39 Uppsala (SE); MOKS, Tomas, S-183 33 Täby (SE)
(72) Inventor: Uhlen, Mathias, S-752 39 Uppsala (SE); MOKS, Tomas, S-183 33 Täby (SE)
(74) Representative: Holmes, Michael John
(86) International application number: SE8900313
(87) International publication number: WO9015148

(56) References cited:
- EP-A- 215 533
- EP-A- 245 945
- GB-A- 2 130 744
- US-A- 4 833 239
- CHEMICAL ABSTRACTS, vol. 110, no. 5, 30 January 1989, Columbus, OH (US); W.F. LIMA et al., no. 37725f#
- ANALYTICAL BIOCHEMISTRY, vol. 148, 1985; D. MICARD et al., pp. 121-126#
- PREPARATIVE BIOCHEMISTRY, vol. 15, no. 3, 1985; J.M. RANHAND, pp. 121-131#
- CHEMICAL ABSTRACTS, vol. 100, no. 5, 30 January 1984, Columbus, OH (US); L. JUST et al., no. 31842p#

## Description

The invention relates to an apparatus and a method for automatic separation of extra-chromosomal DNA from a cell suspension, and is a modification of the apparatus and method described in WO-A 8909265 belonging to the same applicants.

It is known from Birnboim and Doly (Nucl Acids Res 7, 1513-1523, 1979) that plasmid DNA can be purified from Escherichia coli. The bacterial cells are first lysed in an alkaline medium containing a detergent. This is followed by a neutralizing step which yields the plasmid DNA in soluble form while most proteins, cell debris and chromosomal DNA precipitate. Plasmid DNA is then recovered from the mixture by centrifugation and a pure plasmid fraction is obtained by subsequent purification. Kieser (Plasmid 12, 19-36, 1984) has described a similar method, involving a combination of heat treatment and alkali treatment, which gave improved purification of plasmid DNA and less contaminating RNA. In these known methods, the purification involves several separate steps, such as for instance extraction and centrifugation, thus requiring considerable investment in equipment and operator time.

An automated plasmid purification work station based on a laboratory robot has been described by de Bonville and Riedel (Advances in Laboratory Automation Robotics, 1986, ed. Strimatis and Hawk, Zymark Corp., Hopkinton, Ma., pp353-360). That system is based on a number of laboratory unit operations and the robot uses several hardware stations, including a centrifuge, pipettes, capping devices, sample tubes, tip racks, a microtiterplate etc.

Although this work station is automated the system has several disadvantages. Firstly the system has to be set up with tip racks, sample tubes, supernatant tubes etc., which is labour intensive. Secondly, a considerable amount of disposable material is used which increases the expenses for each plasmid purified by the system. Thirdly, several mechanical operations are involved and this might cause technical problems in routine use.

An automated nucleic acid extractor has been described (EP-0 245 945) for extracting and purifying nucleic acids from cells without the use of centrifugation. In the method (described in EP-O 215 533) a lysate is created and mixed with a phenol-based solvent system and then heated to promote phase separation. The lower organic phase is removed and the upper aqueous phase is repeatedly extracted. The aqueous phase is finally dialysed to further purify the nucleic acid solution. The method is used for extraction of high molecular weight DNA (about 10⁸ Daltons) from eukaryotic cells such as blood lymphocytes and liver cells. Thus, the method is well suited for extraction of chromosomal DNA from cells.

With reference to WO-A-8909265, an apparatus and a method for purification of extra-chromosomal DNA from bacteria has been described, which is rapid and avoids phenol extractions and ethanol precipitation. This was attained by a method comprising the following steps:
- introducing the bacterial cell suspension into a chamber,
- introducing a lysing solution into the chamber and mixing it with the contents thereof to lyse the cells therein,
- introducing a precipitating solution into the chamber and mixing it with the contents thereof to precipitate chromosomal DNA and, optionally, proteins and cell debris therein,
- filtering the contents of the chamber through a filter and feeding the liquid contents to a collecting device,
- purifying extra-chromosomal DNA from the contents of the collecting device,
- introducing a dissolving solution into the chamber and mixing it with the contents thereof to dissolve the precipitate remaining therein, and
- feeding the dissolved precipitate from the chamber to waste.

Using this method, a complete cycle from introducing the cell suspension into the chamber to complete regeneration of the chamber and the column took less than ten minutes. A complete purification involving ten samples therefore took approximately ninety minutes under which no supervision or manual labour was needed. The plasmid DNA purified in this way was directly used for restriction analysis and the degree of contamination between different preparations was less than 0.1 percent.

However, the apparatus and the method of WO-A-8909265 have the disadvantage that vacuum and pressurized air are needed for the transport of the cells and to move liquids into and out of the mixing chamber. In addition, the volume of the solutions taken from the solution containers and the volume of the purified DNA solution are determined by the viscosity, time, pressure and/or filter capability. This introduces difficulties in controlling operations in a reproducible manner and makes it necessary to have safety margins which mean that it is difficult to optimize the yield of DNA as well as the time needed for the different operations.

The apparatus and method of the invention, as defined in claims 1 and 17, avoid the need for a vacuum pump and a pressure air source by using a reaction chamber the volume of which is determined by the movement of a piston, thereby allowing the size of the reaction chamber to be controlled by a control unit interfaced with a stepping motor. Solution and cells to by lysed are introduced into the reaction chamber through negative pressure obtained by outward movement of the piston. Filtering is achieved by creating a pressure through inward movement with the piston. Relevant valves must be open or closed during these procedures.

The advantage with the apparatus containing the reaction device as compared to the apparatus described in WO-A-8909265, includes defined sample volumes and defined times for the different operations, which means that less amount of buffers are needed for each cycle and that the cycle time can be further optimized. Also, higher yields are obtained as no safety volumes are needed and the lack of air pressure and vacuum means give safer handling as no containers need to be pressurized. In addition, the volume of the buffers and the cell suspension can be determined by the movement of the piston, which gives a flexible system easily adapted to different host cells.

The reaction chamber and the accompanying apparatus and method according to the invention will be described more in detail below with reference to the accompanying drawings on which
Fig 1 shows a schematic block diagram of an embodiment of an apparatus according to the invention,
Fig 2 shows a drawing of the reaction device consisting of a chamber with a size determined by the position of a movable piston, and
Fig 3 shows an example of the movement of the piston in the reaction device during a separation and regeneration cycle.

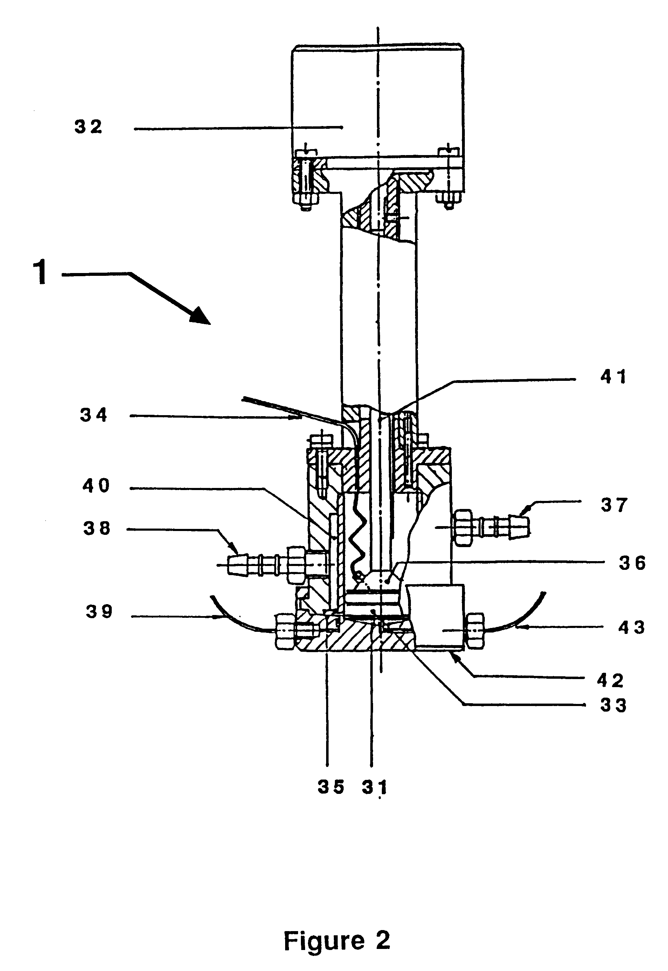

As can be seen from Fig. 2, a reaction and/or mixing chamber 33 with a volume determined by the position of a movable piston 36 is illustrated, comprising a filter with filter support and sealing gasket 31, a stepping motor 32, such as Berger Lahr RPM 564/50, a reaction chamber 33 containing a magnet, capillary tubings 34, 39 and 43, inlet 38 and outlet 37 for cooling and heating water, an O-ring 35, a piston 36, a water jacket for heating/cooling liquid 40, a piston rod with thread 41 and a removable bottom part 42.

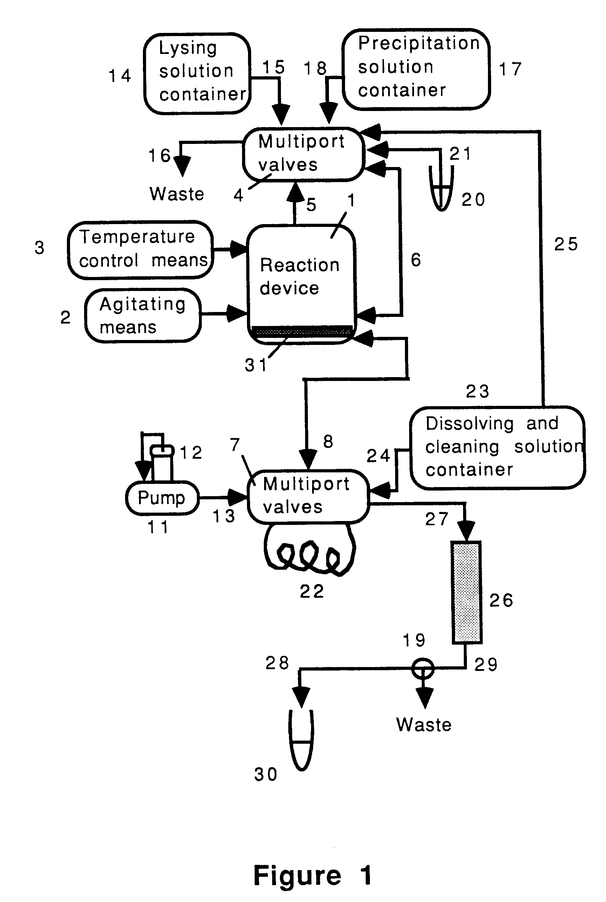

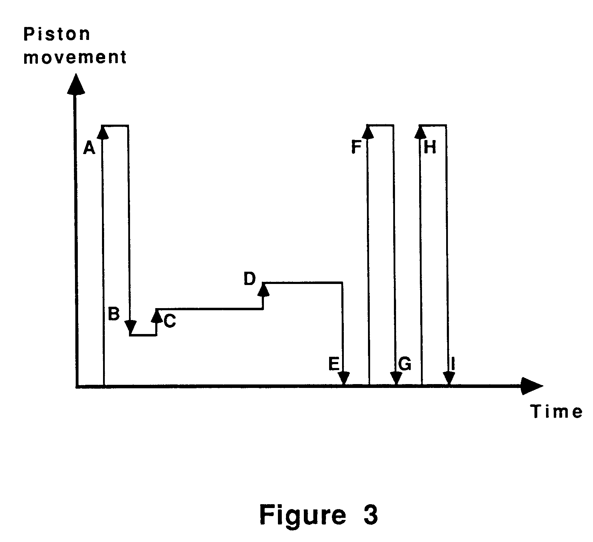

The reaction device operates as follows to automatically separate extra chromosomal DNA from a cell suspension with the numbers refering to the schematic drawing of the complete apparatus in Fig.1 (numbers 1-31) and to the reaction device based on a movable piston, see Fig.2 (numbers 31-43). Note that the filter 31 is shown in Figs 1 and 2 and that the capillary tubings 34, 39 and 43 in Fig. 2 represent the tubings 5, 6 and 8, respectively, in Fig. 1. The movement of liquids in and out of the mixing chamber 33 is obtained through upward and downward respectively, movement of the piston 36, through the action of the stepping motor 32. An example of the motion of the piston is shown in Fig. 3 and will be referred to in the text.

The reaction device 1 is provided with agitating means 2, preferably by said magnet, situated in the reaction chamber 33 between the piston 36 and the filter 31, which is acted on by a magnetic stirrer placed under the reaction device 1 (not shown). The magnet motion can be turn on or off through the control of a control unit. The reaction device 1 is provided with means 3 to control the temperature in the reaction chamber 33. The temperature control can preferably be provided by supplying heating/cooling liquids to a space jacket 40 surrounding the reaction chamber 33. The piston 36 of the reaction device 1 is connected to a stepping motor 32 by a piston rod 41 with a thread, which transfers a rotary movement to the up- and downward motion of the the piston 36. The volume of the reaction chamber 33 is determined by the position of the piston. The reaction chamber 33 may be provided with temperature sensor means (not shown) to sense the temperature inside the reaction chamber 33.

At the bottom of the reaction chamber 33, a filter 31 is provided. This filter may consist of material such as metal or polymer.

An inlet/outlet port is connected to an outlet/inlet port of multiport valves 4 via a tubing 39 (6) , while an outlet port in the upper part of the reaction chamber is connected via a tubing 34 (5) to an inlet port of the multiport valves 4. A pressure valve may be provided to tubing 34 (5) to prevent high pressures in the reaction chamber 33 to be built up during filtration. An outlet/inlet port at the bottom of the reaction device 1 is connected to an inlet/outlet port of the multiport valves 7 via a tubing 43 (8).

The multiport valves 4 have an outlet port connected to waste via a tubing 16. Moreover, the multiport valves 4 are connected to a precipitating solution container 17 via a tubing 18. Another inlet port of the multiport valves 4 is connected to a lysing solution container 14 via a tubing 15.

A tube or vial 20 containing a cell suspension from which extra-chromosomal DNA is to be separated may be connected to an inlet port of the multiport valves 4 via an automatic injector or sampler 21, which is just schematically indicated. It is to be understood that there can be a number of vials 20 from which extra-chromosomal DNA is to separated in sequential order.

A sample is sucked into the reaction chamber 33 through upward movement of the piston 36 using the step motor 32. The volume of cell suspension drawn into the chamber 33 is determined by the movement of the piston 36 (see Fig. 3).

The multiport valves 7 have an inlet port and an outlet port between which a collecting device in the form of a sample loop 22 of predetermined volume is connected in the embodiment shown. A container 23 containing a dissolving and cleaning solution is connected to an inlet port of the multiport valves 4 and 7 via tubings 25 and 24, respectively.

The inlet end of a chromatography gel filtration column 26 may be connected to an outlet port of the multiport valves 7 via a tubing 27, while the outlet end of the column 26 is connected to a drip nozzle 28 of a fraction collector (not shown) via a tubing 29. Drops of purified extra-chromosomal DNA from the drip nozzle 28 are collected in a vial 30 of the fraction collector. It is to be understood that there can be a number of vials 30 to collect extra-chromosomal DNA extracted in sequence from the cell suspension vials 20.

In the tubings between the outlet end of the column 26 and the drip nozzle 28 of the fraction collector (not shown), a two-way valve 19 is inserted to connect the outlet end of the column 26 to either the drip nozzle 28 or waste.

An eluant for the chromatography column 26 is supplied by a pump 11 from an eluant container 12 via a tubing 13 to an inlet port of the multiport valves 7.

The multiport valves 4 and 7 as well as the agitating means 2, the temperature control means 3, the step motor 32, the sampler 21, the pump 11 and the fraction collector (symbolized by 28 and 30) are all controlled by a control unit (not shown) in a manner known per se.

The apparatus shown on the drawing (Fig. 1) having the reaction device shown in Fig. 2 operates as follows to automatically separate extra-chromosomal DNA from a cell suspension in the vial 20:
The cell suspension is sucked from the vial 20 into the reaction and/or mixing chamber 33 using an upward movement of the piston 36 as schematically shown in Fig. 3 (A). Thus a predetermined volume of the cell suspension is sucked up by the sampler 21 and enters the mixing chamber through the tubing 6 (39) via the multiport valves 4.

The cell suspension may, optionally, be concentrated by filtering the suspension through the filter 31 (Fig.2) through downward movement of piston 36 as schematically illustrated in Fig.3 (B). The reaction chamber is, optionally, agitated during the filtration by means of the magnet placed in the chamber 33.

The cells in the concentrated cell suspension are then lysed; thus a predetermined amount of lysing solution from the lysing solution container 14 is introduced into the reaction chamber 33 using upward movement of the piston 36 (Fig.3, C) via the tubing 15, the multiport valves 4 and the tubing 6 (39). The mixture is mixed using the agitating means 2. Optionally, the reaction chamber is heated by means of the temperature control means 3, which is achieved by introducing preheated liquid into the jacket 40 via the inlet 38 and the outlet 37.

To precipitate chromosomal DNA, proteins and cell debris from the content of the reaction chamber, a predetermined amount of precipitating solution from the precipitating solution container 17 is then introduced into the rection chamber using upward movement of the piston 36 (Fig.3, D) via the tubing 18, the multiport valves 4 and the tubing 6 (39).

The mixture is agitated using the agitating means 2 and, optionally, the reaction chamber is cooled by means of temperature control means 3. This is achieved by introducing cooling liquid into the jacket 40 via inlet 38 and the outlet 46.

The contents of the reaction chamber 33 are then filtered through the filter 31 by means of pressure caused by downward movement of the piston 36 (Fig.3, E). A predetermined volume of the liquid content of the reaction chamber 33 is collected the loop 22 via the tubing 8 (43) and the multiport valves 7, while precipitated chromosomal DNA, proteins and cell debris will remain on the filter 31.

Where it is desired to purify the separated extra-chromosomal DNA, the contents of the loop 22 may be applied to the chromatographic column 26 by means of the pump 11. Impure fractions are supplied to waste by the valve 19, while pure extra-chromosomal DNA is collected in the vial 30 from the drip nozzle 28.

At the same time as the chromatographic purification takes place the precipitate remaining on the filter 31 and within the reaction chamber 33 is dissolved; thus a predetermined amount of dissolving and cleaning solution from the container 23 is introduced by upward movement of the piston 36 (Fig.3, F) into the reaction chamber 33 through the tubing 25, the multiport valves 4 and the tubing 6 (39).

After agitating the reaction chamber 33 by means of the agitating means to dissolve and loosen all the precipitate and, optionally by heating the chamber by introducing heating water into the jacket 40 using the inlet 38 and the outlet 37, the dissolved precipitate is passed to waste by means of the pressure caused by downward movement of the piston 36 (Fig 3, G) via the tubings 6 (39), the multiport valves 4 and the tubing 16.

This procedure can be repeated once (Fig.3, H and I) or several times (not shown). The reaction chamber 33 is then ready to receive another cell suspension from the sampler 21 to start a new separation and, if desired, purification cycle.

Instead of the column 26, means can be provided to add a DNA precipitating agent to the contents of the loop 22 to purify the separated extra-chromosomal precipitation in a manner known per se.

The means for purifying extra-chromosomal DNA may also comprise adsorbing means, e.g. a solid adsorption matrix, for adsorbing purified extra-chromosomal DNA from the contents of the loop 22.

The purification mentioned in the above two paragraphs is suitably carried out in a separate chaser or container (not shown).

## Claims

1. Apparatus for automated separation of extra-chromosomal DNA from a cell suspension comprising a reaction chamber (33) having a filter (31) provided therein and inlet/outlet ports (39, 43) communicating with said chamber (33) on opposite sides of said filter (31), characterised in that the chamber (33) on one side of said filter (31) has a piston (36) moveable therein to change the volume thereof.

2. Apparatus as claimed in claim 1 wherein the inlet/outlet port (39) on the piston side of filter (31) is connected to means adapted for the successive introduction of a cell suspension, a lysing solution, a precipitating solution and a dissolving and cleaning solution.

3. Apparatus as claimed in claim 1 or claim 2 incorporating agitating means on the piston side of filter (31).

4. Apparatus as claimed in claim 3 wherein said agitating means comprise a magnetic stirrer.

5. Apparatus as claimed in any of the preceding claims wherein movement of the piston (36) is controlled by a stepping motor (32) connected thereto by way of a threaded piston rod (41).

6. Apparatus as claimed in any of the preceding claims incorporating temperature control means.

7. Apparatus as claimed in claim 6 wherein said temperature control means comprise an enclosing jacket (40) through which heating and/or cooling liquids may be passed through inlet (38) and outlet (37).

8. Apparatus as claimed in any of the preceding claims wherein an outlet (34) connects with the chamber (33) by way of a bore passing through piston (36).

9. Apparatus as claimed in claim 8 wherein said outlet (34) is connected to pressure regulating or sensing means.

10. Apparatus as claimed in any of the preceding claims wherein the inlet/outlet port (43) on the side of filter (31) remote from piston (36) connects with a sample collecting loop (22) of predetermined or controllably variable volume.

11. Apparatus as claimed in claim 10 wherein said sample loop (22) is further connected to purifying means (26) adapted to purify extra-chromosomal DNA.

12. Apparatus as claimed in claim 11 wherein said purifying means (26) comprise a chromatographic gel filtration column.

13. Apparatus as claimed in claim 12 wherein said column is additionally provided with means for introducing eluant or dissolving and cleaning solution to the column.

14. Apparatus as claimed in claim 11 wherein said purifying means (26) comprise means for adding a DNA precipitating agent.

15. Apparatus as claimed in claim 11 wherein said purifying means (26) comprise means for adsorbing extra-chromosomal DNA.

16. Apparatus as claimed in claim 15 wherein said adsorbing means comprise a solid adsorption matrix.

17. A method for automated separation of extra-chromosomal DNA using apparatus as claimed in claim 1, in which a cell suspension, a lysing solution and a precipitating solution are successively drawn into reaction chamber (33) through inlet/outlet port (39) by movement of piston (36), whereby there are successively effected lysis of the cells and precipitation of chromosomal DNA, optionally together with proteins and cell debris, whereafter the resulting liquid containing extra-chromosomal DNA is passed to a collection device through precipitate-retaining filter (31) and inlet/outlet port (43) by movement of piston (36).

18. A method as claimed in claim 17 wherein dissolving and cleaning solution is subsequently drawn into and thereafter removed from reaction chamber (33) through at least one of the inlet/outlet ports (39, 43) by movement of piston (36).

19. A method as claimed in claim 17 or claim 18 wherein the cell suspension is concentrated prior to addition of the lysing solution by movement of piston (36) to drive superfluous liquid through the filter (31).

20. A method as claimed in any of claims 17 to 19 wherein the reaction chamber (33) is heated during lysis of the cells.

21. A method as claimed in any of claims 17 to 20 wherein the reaction chamber (33) is cooled during precipitation of chromosomal DNA.

## Patentansprüche

1. Vorrichtung zur automatischen Trennung von extra-chromosomaler DNA von einer Zellsuspension, umfassend eine Reaktionskammer (33) mit einem darin vorgesehenen Filter (31) und Einlaß/Auslaß-Öffnungen (39,43), die mit der Kammer (33) auf gegenüberliegenden Seiten des Filters (31) in Verbindung stehen,
**dadurch gekennzeichnet,**
daß die Kammer (33) auf einer Seite des Filters (31) einen Kolben (36) aufweist, der in der Kammer zum Ändern von deren Volumen beweglich ist.

2. Vorrichtung nach Anspruch 1, wobei die Einlaß/Auslaß-Öffnung (39) auf der Kolbenseite des Filters (31) an eine Einrichtung angeschlossen ist, mittels der aufeinanderfolgend eine Zellsuspension, eine lysierende Lösung, eine Ausfällösung und eine Auflösungs- und Reinigungslösung eingeleitet werden kann.

3. Vorrichtung nach Anspruch 1, mit einer Rühreinrichtung auf der Kolbenseite des Filters (31).

4. Vorrichtung nach Anspruch 3, wobei die Rühreinrichtung einen magnetischen Rührer umfaßt.

5. Vorrichtung nach einem der vorhergehenden Ansprüche, wobei die Bewegung des Kolbens (36) durch einen Schrittmotor (32) gesteuert wird, der mit diesem über eine mit einem Gewinde versehene Kolbenstange (41) verbunden ist.

6. Vorrichtung nach einem der vorhergehenden Ansprüche, umfassend eine Temperatursteuereinrichtung.

7. Vorrichtung nach Anspruch 6, wobei die Temperatursteuereinrichtung einen umhüllenden Mantel (40) aufweist, durch den heizende und/oder kühlende Flüssigkeiten über den Einlaß (38) und den Auslaß (37) geleitet werden.

8. Vorrichtung nach einem der vorhergehenden Ansprüche, wobei ein Auslaß (34) mit der Kammer (33) über eine Bohrung verbunden ist, die durch den Kolben (36) verläuft.

9. Vorrichtung nach Anspruch 8, wobei der Auslaß (34) mit einer Druckregulierungseinrichtung oder einer Druckabtasteinrichtung verbunden ist.

10. Vorrichtung nach einem der vorhergehenden Ansprüche, wobei die Einlaß/Auslaß-Öffnung (43) auf der Seite des Filters (31) vom Kolben (36) abliegend mit einer Schleife (22) von vorbestimmtem oder steuerbarem, variablem Volumen zur Aufnahme einer Probe verbunden ist.

11. Vorrichtung nach Anspruch 10, wobei die Probenschleife (22) an eine Reinigungseinrichtung (26) angeschlossen ist, mittels der extra-chromosomale DNA gereinigt werden kann.

12. Vorrichtung nach Anspruch 11, wobei die Reinigungseinrichtung (26) eine Filtersäule mit einem chromatographischen Gel umfaßt.

13. Vorrichtung nach Anspruch 12, wobei die Säule zusätzlich mit einer Einrichtung zum Einleiten eines Extraktionsmittels oder einer Auflösungs- und Reinigungslösung in die Säule versehen ist.

14. Vorrichtung nach Anspruch 11, wobei die Reinigungseinrichtung (26) eine Einrichtung für die Zugabe eines Mittels zum Ausfällen der DNA umfaßt.

15. Vorrichtung nach Anspruch 11, wobei die Reinigungseinrichtung (26) eine Einrichtung zum Adsorbieren extra-chromosomaler DNA umfaßt.

16. Vorrichtung nach Anspruch 15, wobei die adsorbierende Einrichtung eine feste Adsorptionsmatrix umfaßt.

17. Verfahren zum automatischen Trennen von extra-chromosomaler DNA unter Verwendung einer Vorrichtung nach Anspruch 1, wobei eine Zellsuspension, eine lysierende Lösung und eine Ausfällösung nacheinander in die Reaktionskammer (33) über die Einlaß/Auslaß-Öffnung (39) durch die Bewegung des Kolbens (36) eingezogen werden, wodurch darin nacheinander die Lyse der Zellen und die Ausfällung von chromosomaler DNA, gegebenenfalls zusammen mit Proteinen und Zelldebris bewirkt wird, wonach die sich ergebende Flüssigkeit, die extra-chromosomale DNA enthält, zu einer Sammelvorrichtung über ein Ausfäll-Rückhaltefilter (31) und eine Einlaß/Auslaß-Öffnung (43) durch die Bewegung des Kolbens (36) geleitet wird.

18. Verfahren nach Anspruch 17, wobei die auflösende und reinigende Lösung anschließend in die Reaktionskammer (33) eingezogen und danach aus dieser durch wenigstens eine der Einlaß/Auslaß-Öffnungen (39,43) durch die Bewegung des Kolbens (36) abgezogen wird.

19. Verfahren nach Anspruch 17 oder 18, wobei die Zellsuspension vor der Zugabe der lysierenden Lösung durch die Bewegung des Kolbens (36) konzentriert wird, indem überflüssige Flüssigkeit durch das Filter (31) gedrückt wird.

20. Verfahren nach einem der Ansprüche 17 bis 19, wobei die Reaktionskammer (33) während der Lyse der Zellen erwärmt wird.

21. Verfahren nach einem der Ansprüche 17 bis 20, wobei die Reaktionskammer (33) während der Ausfällung chromosomaler DNA gekühlt wird.

## Revendications

1. Appareil de séparation automatisée de l'ADN extrachromosomique d'une suspension de cellules, qui comprend une chambre de réaction (33) possédant un filtre (31) qui s'y trouve logé et des ouvertures d'entrée/sortie (39, 43) communiquant avec cette chambre (33) par des côtés opposés du filtre précité (31), caractérisé en ce que la chambre (33) comporte, d'un côté du filtre précité (31) un piston (36) qui y est mobile pour en changer le volume.

2. Appareil suivant la revendication 1, caractérisé en ce que l'ouverture d'entrée/sortie (39) du côté piston du filtre (31) est raccordée à un dispositif adapté à l'introduction successive d'une suspension de cellules, d'une solution de lyse, d'une solution de précipitation et d'une solution de dissolution et de nettoyage.

3. Appareil suivant la revendication 1 ou la revendication 2, comprenant un dispositif d'agitation du côté piston du filtre (31).

4. Appareil suivant la revendication 3, caractérisé en ce que le dispositif d'agitation comprend un agitateur magnétique.

5. Appareil suivant l'une quelconque des revendications précédentes, caractérisé en ce que le mouvement du piston (36) est commandé par un moteur pas-à-pas (32) qui est raccordé à l'aide d'une tige de piston filetée (41).

6. Appareil suivant l'une quelconque des revendications précédentes, auquel est incorporé un dispositif de réglage ou de commande de la température.

7. Appareil suivant la revendication 6, caractérisé en ce que le dispositif de réglage ou de commande de la température comprend une chemise enveloppante (40) à travers laquelle on peut faire circuler des liquides de chauffage et/ou de refroidissement par l'entrée (38) et la sortie (37).

8. Appareil suivant l'une quelconque des revendications précédentes, caractérisé en ce qu'une sortie (34) se raccorde à la chambre (33) par l'intermédiaire d'un trou percé passant à travers le piston (36).

9. Appareil suivant la revendication 8, caractérisé en ce que la sortie (34) est raccordée à un dispositif capteur ou régulateur de pression.

10. Appareil suivant l'une quelconque des revendications précédentes, caractérisé en ce que l'ouverture d'entrée/sortie (43) du côté du filtre (31) éloigné du piston (36) est raccordé à un circuit collecteur d' échantillon (22) de volume prédéterminé ou variable sous commande.

11. Appareil suivant la revendication 10, caractérisé en ce que le circuit d'échantillon (22) précité est également raccordé à un dispositif d'épuration (26) adapté à purifier l'ADN extrachromosomique.

12. Appareil suivant la revendication 11, caractérisé en ce que le dispositif épurateur (26) précité comprend une colonne de filtration sur gel chromatographique.

13. Appareil suivant la revendication 12, caractérisé en ce que ladite colonne est, de surcroît, pourvue d'un dispositif pour introduire un éluant ou dissoudre et nettoyer la solution dans la colonne.

14. Appareil suivant la revendication 11, caractérisé en ce que le dispositif épurateur (26) comprend un dispositif pour ajouter un agent de précipitation de l'ADN.

15. Appareil suivant la revendication 11, caractérisé en ce que le dispositif épurateur (26) comprend un dispositif pour adsorber l'ADN extrachromosomique.

16. Appareil suivant la revendication 15, caractérisé en ce que le dispositif adsorbeur comprend une matrice d'adsorption solide.

17. Procédé de séparation automatisée d'ADN extrachromosomique utilisant l'appareil suivant la revendication 1, caractérisé en ce qu'une suspension de cellules, une solution de lyse et une solution de précipitation sont successivement tirées ou aspirées dans la chambre de réaction (33) par l'ouverture d'entrée/sortie (39) sous l'effet du mouvement du piston (36), si bien que s'effectuent successivement la lyse des cellules et la précipitation de l'ADN chromosomique, éventuellement en même temps que de protéines et de débris cellulaires, puis le liquide résultant qui contient de l'ADN extrachromosomique est envoyé dans un dispositif collecteur à travers un filtre (31) retenant le précipité et l'ouverture d'entrée/sortie (43) sous l'effet du mouvement du piston (36).

18. Procédé suivant la revendication 17, caractérisé en ce que l'on tire ou aspire subséquemment la solution de dissolution et de nettoyage dans la chambre de réaction (33) et qu'on l'en enlève à travers au moins l'une des ouvertures d'entrée/sortie (39, 43) sous l'effet du mouvement du piston (36).

19. Procédé suivant la revendication 17 ou la revendication 18, caractérisé en ce que l'on concentre la suspension de cellules préalablement à l'addition de la solution de lyse sous l'effet du mouvement du piston (36) pour chasser le liquide superflu à travers le filtre (31).

20. Procédé suivant l'une quelconque des revendications 17 à 19, caractérisé en ce que l'on chauffe la chambre (33) au cours de la lyse des cellules.

21. Procédé suivant l'une quelconque des revendications 17 à 20, caractérisé en ce que l'on refroidit la chambre de réaction (33) au cours de la précipitation de l'ADN chromosomique.
